(19) Europäisches Patentamt / European Patent Office / Office européen des brevets

(11) **EP 3 449 960 A2**

(12) **EUROPÄISCHE PATENTANMELDUNG**

(43) Veröffentlichungstag:
**06.03.2019 Patentblatt 2019/10**

(51) Int Cl.:
***A61M 1/36*** *(2006.01)*

(21) Anmeldenummer: **18196769.6**

(22) Anmeldetag: **27.07.2012**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.07.2011 DE 102011108777**
**29.07.2011 US 201161512930 P**

(62) Dokumentnummer(n) der früheren Anmeldung(en) nach Art. 76 EPÜ:
**12743386.0 / 2 736 560**

(71) Anmelder: **Fresenius Medical Care Deutschland GmbH**
**61352 Bad Homburg (DE)**

(72) Erfinder:
• **Gronau, Soeren**
**65428 Rüsselsheim (DE)**

• **Haecker, Juergen**
**61267 Neu-Anspach (DE)**
• **Mueller, Ralf**
**61350 Bad Homburg (DE)**
• **Weis, Manfred**
**66606 St. Wendel (DE)**
• **Kreber, Stefan**
**66113 Saarbrücken (DE)**
• **Thys, Martin**
**71116 Gärtingen (DE)**

(74) Vertreter: **Bobbert & Partner**
**Patentanwälte PartmbB**
**Postfach 1252**
**85422 Erding (DE)**

Bemerkungen:
Diese Anmeldung ist am 26.09.2018 als Teilanmeldung zu der unter INID-Code 62 erwähnten Anmeldung eingereicht worden.

(54) **VERFAHREN ZUM ENTFERNEN VON BLUT AUS EINEM EXTRAKORPORALEN BLUTKREISLAUF SOWIE VORRICHTUNGEN**

(57) Die vorliegende Erfindung betrifft ein Verfahren zum Entfernen von Blut aus einem extrakorporalen Blutkreislauf (2000) und/oder einer Funktionseinrichtung (1000), welche jeweils zum Zweck einer Blutbehandlung eines Patienten mit einer Blutbehandlungsvorrichtung verbindbar oder verbunden sind, nach Beenden der Blutbehandlungssitzung, wobei die Blutbehandlungsvorrichtung aufweist oder verbunden ist mit wenigstens einen/m extrakorporalen Blutkreislauf (2000) mit einem Leitungsinneren, wobei der extrakorporale Blutkreislauf (2000) wenigstens einen arteriellen Leitungsabschnitt (1) und wenigstens einen venösen Leitungsabschnitt (3) aufweist; wenigstens eine/r Blutpumpe (4000) zum Fördern von Blut innerhalb des Leitungsinneren, wobei das Verfahren den Schritt des Betreibens der Blutpumpe (4000) in einer der während der Blutbehandlung üblichen ersten Förderrichtung entgegengesetzten zweiten Förderrichtung umfasst, wobei ein erster Abschnitt des arteriellen Leitungsabschnitts (1) mit einem zweiten Abschnitt des venösen Leitungsabschnitts (3) des extrakorporalen Blutkreislaufs (2000) verbunden ist oder wird. Sie betrifft ferner entsprechende Vorrichtungen.

Fig. 1

**Beschreibung**

Die vorliegende Erfindung betrifft ein hierin beschriebenes Verfahren zum Entfernen von Blut aus einem extrakorporalen Blutkreislauf und/oder aus einer Funktionseinrichtung, welche jeweils zum Zweck einer Blutbehandlung eines Patienten mit einer Blutbehandlungsvorrichtung verbindbar oder verbunden ist bzw. sind. Zudem betrifft sie eine Funktionseinrichtung gemäß Anspruch 1 sowie ein Set gemäß Anspruch 2. Die Erfindung betrifft ferner eine hierin beschriebene Steuereinrichtung sowie eine Blutbehandlungsvorrichtung gemäß Anspruch 3. Des Weiteren betrifft die vorliegende Erfindung ein digitales Speichermedium, ein Computerprogramm-Produkt sowie ein Computerprogramm.

Aus extrakorporalen Blutkreisläufen wird nach ihrem Einsatz im Blutkreislauf verbliebenes Blut nicht zuletzt aus Hygienegründen regelmäßig aus diesem entfernt.

Eine Aufgabe der vorliegenden Erfindung ist es, ein weiteres Verfahren zum Entfernen von Blut aus einem extrakorporalen Blutkreislauf oder aus einer Funktionseinrichtung nach Beenden einer Blutbehandlungssitzung anzugeben.

Ferner sollen eine Blutbehandlungsvorrichtung, mit welcher das erfindungsgemäße Verfahren durchführbar ist sowie eine zur Durchführung des erfindungsgemäßen Verfahrens vorgesehene Steuereinrichtung, ein geeignetes digitales Speichermedium, ein geeignetes Computerprogramm-Produkt und ein geeignetes Computerprogramm angegeben werden.

Die erfindungsgemäße Aufgabe wird durch ein hierin beschriebenes Verfahren gelöst. Zudem wird sie gelöst durch eine Funktionseinrichtung mit den Merkmalen des Anspruchs 1 sowie durch ein Set mit den Merkmalen des Anspruchs 2. Sie wird ferner durch eine hierin beschriebene Steuereinrichtung sowie eine Blutbehandlungsvorrichtung mit den Merkmalen des Anspruchs 3 gelöst. Die erfindungsgemäße Aufgabe wird ferner gelöst durch ein digitales Speichermedium, ein Computerprogramm-Produkt sowie ein Computerprogramm wie jeweils hierin beschrieben.

Alle mit dem erfindungsgemäßen Verfahren erzielbaren Vorteile lassen sich in bestimmten erfindungsgemäßen Ausführungsformen ungeschmälert auch mit den erfindungsgemäßen Vorrichtungen erzielen.

Das erfindungsgemäße Verfahren ist geeignet und vorgesehen zum Entfernen von Blut aus einem extrakorporalen Blutkreislauf, welcher wenigstens einen arteriellen Leitungsabschnitt und wenigstens einen venösen Leitungsabschnitt aufweist, und/oder zum Entfernen von Blut aus einer Funktionseinrichtung, welche zum Zweck einer Blutbehandlung eines Patienten mit einer Blutbehandlungsvorrichtung verbindbar oder verbunden ist bzw. sind, oder jeweils aus Abschnitten hiervon.

Das erfindungsgemäße Verfahren umfasst das Fördern des im extrakorporalen Blutkreislauf nach Beenden der Blutbehandlungssitzung noch vorliegenden Fluids mittels einer Blutpumpe, welche bereits zur Blutbehandlung verwendet wurde. Während die Blutpumpe bei der Blutbehandlung in einer ersten Förderrichtung gefördert hat, wird mittels der Blutpumpe beim erfindungsgemäßen Verfahren in einer zweiten, der ersten Förderrichtung entgegengesetzten Förderrichtung gefördert. Zur Durchführung des erfindungsgemäßen Verfahrens wird oder ist ein erster Abschnitt des arteriellen Leitungsabschnitts mit einem zweiten Abschnitt des venösen Leitungsabschnitts des extrakorporalen Blutkreislaufs verbunden.

Die erfindungsgemäße Funktionseinrichtung, welche insbesondere als Blutkassette ausgestaltet ist, weist einen venösen Leitungsabschnitt und eine hiermit in Verbindung stehende venöse Zugabestelle auf. Die venöse Zugabestelle ist vorbereitet und/oder vorgesehen, um mit einem arteriellen Nadelanschluss des arteriellen Leitungsabschnitts eines extrakorporalen Blutkreislaufs konnektiert zu werden.

Das erfindungsgemäße Set weist wenigstens eine erfindungsgemäße Funktionseinrichtung und wenigstens einen extrakorporalen Blutkreislauf auf. Der arterielle Nadelanschluss oder ein anderer Abschnitt des arteriellen Leitungsabschnitts des extrakorporalen Blutkreislaufs ist vorbereitet, um mit der venösen Zugabestelle der Funktionseinrichtung konnektiert zu werden.

Die erfindungsgemäße Blutbehandlungsvorrichtung ist vorgesehen und ausgestaltet und/oder ausgestattet zur Durchführung des erfindungsgemäßen Verfahrens.

Die erfindungsgemäße Steuereinrichtung ist geeignet und vorgesehen und/oder ausgelegt und/oder konfiguriert zur Durchführung des erfindungsgemäßen Verfahrens.

Ein erfindungsgemäßes digitales, insbesondere nichtflüchtiges, Speichermedium, insbesondere in Form eines maschinenlesbaren Trägers, insbesondere in Form einer Diskette, CD, EPROM oder DVD, mit elektrisch auslesbaren Steuersignalen kann derart mit einem programmierbaren Computersystem zusammenwirken, dass die maschinellen Schritte eines erfindungsgemäßen Verfahrens veranlasst werden.

Dabei können alle, einige oder manche der maschinell durchgeführten Schritte des erfindungsgemäßen Verfahrens veranlasst werden.

Ein erfindungsgemäßes Computerprogramm-Produkt weist einen volatilen, flüchtigen oder auf einem maschinenlesbaren Träger gespeicherten Programmcode zur Veranlassung der maschinellen Schritte des erfindungsgemäßen Verfahrens, wenn das Computerprogramm-Produkt auf einem Rechner abläuft, auf. Unter einem Computerprogramm-Produkt kann erfindungsgemäß beispielsweise ein auf einem Träger gespeichertes Computerprogramm, ein Embedded System als umfassendes System mit einem Computerprogramm (z. B. elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte oder dergleichen sein.

Ein erfindungsgemäßes Computerprogramm weist einen Programmcode auf zur Veranlassung der maschinellen Schritte eines erfindungsgemäßen Verfahrens, wenn das Computerprogramm auf einem Computer abläuft. Unter einem Computerprogramm kann erfindungsgemäß beispielsweise ein physikalisches, vertriebsfähiges Software-Produkt verstanden werden, welches ein Programm aufweist.

Auch für das erfindungsgemäße Computerprogramm-Produkt und das erfindungsgemäße Computerprogramm gilt, dass alle, einige oder manche der maschinell durchgeführten Schritte des erfindungsgemäßen Verfahrens veranlasst werden.

Erfindungsgemäße Ausführungsformen können manche, einige oder alle der folgenden Merkmale in beliebiger Kombination aufweisen. Vorteilhafte Weiterbildungen der vorliegenden Erfindung sind jeweils auch Gegenstand der Unteransprüche.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Der extrakorporale Blutkreislauf ist in bestimmten Ausführungsformen der vorliegenden Erfindung ein Schlauchset. In jedem Fall ist der extrakorporale Blutkreislauf vorgesehen zum extrakorporalen Leiten von Blut eines Patienten, beispielsweise bei der Hämodialyse, der Hämofiltration, der Hämodiafiltration oder dergleichen.

In einigen erfindungsgemäßen Ausführungsformen ist der extrakorporale Blutkreislauf zumindest abschnittsweise als integraler und ggf. unlösbarer Bestand der Funktionseinrichtung ausgestaltet, in anderen nicht. So kann sich ein frei beweglicher Schlauchabschnitt des extrakorporalen Blutkreislaufs in einem einstückig oder integral auf oder in der Funktionseinrichtung, beispielsweise einer Blutkassette, fortsetzen und umgekehrt.

Eine Funktionseinrichtung ist in bestimmten Ausführungsformen der vorliegenden Erfindung eine Einrichtung, welche bei einer Blutbehandlung verwendet wird. Beispiele für Funktionseinrichtungen schließen interne und externe Funktionseinrichtungen, medizinische Disposables, insbesondere Blutkassetten wie eine Einmal-Blutkassette, oder andere blutführende Einrichtungen ein.

Beispielhafte Ausführungsformen einer Blutkassette sind insbesondere in der Anmeldung der Anmelderin mit der Veröffentlichungsnummer DE 10 2009 018 664 A1 mit dem Titel *"Externe Funktionseinrichtung, Blutbehandlungsvorrichtung zum Aufnehmen einer erfindungsgemäßen externen Funktionseinrichtung, sowie Verfahren",* die am 23.04.2009 beim Deutschen Patent- und Markenamt eingereicht wurde, und in der Anmeldung der Anmelderin mit der Veröffentlichungsnummer DE 10 2009 024 468 A1 mit demselben Titel, die am 10.06.2009 beim Deutschen Patent- und Markenamt eingereicht wurde, offenbart. Auf die diesbezüglichen Offenbarungen dieser beiden Anmeldungen wird hiermit jeweils vollinhaltlich Bezug genommen.

In bestimmten erfindungsgemäßen Ausführungsformen ist die Funktionseinrichtung als externe Funktionseinrichtung kein Teil der Blutbehandlungsvorrichtung, d h. kein integrales Bauteil derselben. In anderen Ausführungsformen kann die Funktionseinrichtung Teil der Blutbehandlungsvorrichtung sein.

Eine Blutbehandlungsvorrichtung ist dazu vorgesehen und/oder ausgestaltet, eine medizinische Behandlung, insbesondere eine Blutbehandlung des Patienten, z. B. eine Dialyse, auszuführen oder zu veranlassen. Hierzu ist die Blutbehandlungsvorrichtung mit wenigstens einem extrakorporalen Blutkreislauf, welcher ein Leitungsinneres aufweist, verbunden oder weist einen solchen auf.

Der arterielle Leitungsabschnitt des extrakorporalen Blutkreislaufs ist in bestimmten Ausführungsformen der vorliegenden Erfindung jener Leitungsabschnitt, in welchen das den Patientenkörper zum Zwecke der extrakorporalen Blutbehandlung verlassende Patientenblut einströmt und in welchem es sich vor Eintritt in die Blutbehandlungseinrichtung, z. B. einen Dialysator, befindet.

In bestimmten Ausführungsformen der vorliegenden Erfindung ist oder umfasst der erste Abschnitt des arteriellen Leitungsabschnitts den arteriellen Nadelanschluss zum Patienten, z. B. den arteriellen Nadelanschluss bei einem Double-Needle-Dialyseverfahren.

Der venöse Leitungsabschnitt des extrakorporalen Blutkreislaufs ist in manchen Ausführungsformen der vorliegenden Erfindung jener Leitungsabschnitt, aus welchem das extrakorporal behandelte Patientenblut nach seiner Behandlung in einer Blutbehandlungseinrichtung, beispielsweise einem Dialysator, zum Patientenkörper hin oder in diesen zurückströmt.

In bestimmten Ausführungsformen der vorliegenden Erfindung ist oder umfasst der zweite Abschnitt des venösen Leitungsabschnitts einen venösen Port, beispielsweise einen venösen Zugabeport oder eine venöse Zugabestelle. Dieser kann ggf. auch genutzt werden oder vorgesehen sein zur Zugabe von Substituatflüssigkeit, Calciumcitrat, Heparin oder dergleichen zu dem im venösen Leitungsabschnitt des extrakorporalen Blutkreislaufs strömenden Patientenblut.

In bestimmten Ausführungsformen der vorliegenden Erfindung mündet die venöse Zugabestelle des venösen Leitungsabschnitts des extrakorporalen Blutkreislaufs stromaufwärts einer Blutkammer und stromaufwärts eines Gerinnselfängers, optional direkt oder indirekt, in den venösen Leitungsabschnitt.

In manchen erfindungsgemäßen Ausführungsformen ist die venöse Zugabestelle integraler Teil einer Blutkassette.

Die Blutbehandlungsvorrichtung weist eine Blutpumpe zum Fördern von Blut innerhalb des Leitungsinneren des extrakorporalen Blutkreislaufs auf.

In bestimmten Ausführungsformen der vorliegenden Erfindung entspricht die - während der Blutbehandlung übliche - erste Förderrichtung einer Förderrichtung vom arteriellen Zugang (Blutentnahmestelle) vom Patienten zu einer Blutbehandlungseinrichtung, beispielsweise einem Blutfilter oder einem Dialysator, und anschließend durch den venösen Leitungsabschnitt des extrakorporalen Blutkreislaufs zum venösen Zugang (Blutrückgabestelle).

Die der ersten Förderrichtung entgegengesetzte zweite Förderrichtung verläuft daher in solchen Ausführungsformen von venös nach arteriell.

Ein "Entfernen von Blut" bezeichnet in bestimmten Ausführungsformen der vorliegenden Erfindung ein vollständiges - oder im Wesentlichen oder nahezu vollständiges - oder ein teilweises Entfernen von Blut aus dem extrakorporalen Blutkreislauf nach Beenden einer Blutbehandlungssitzung.

Ein Reinfundieren des aus dem extrakorporalen Blutkreislauf entfernten Bluts in das Gefäßsystem des Patienten selbst ist in einigen Ausführungsformen der vorliegenden Erfindung nicht Teil des erfindungsgemäßen Verfahrens, in anderen hingegen schon.

In bestimmten Ausführungsformen der vorliegenden Erfindung weist die Blutbehandlungsvorrichtung wenigstens eine zweite Fördereinrichtung auf. Die zweite Fördereinrichtung dient dem Einbringen wenigstens eines zweiten, vom Blut verschiedenen Fluids, beispielsweise einer Substituatflüssigkeit, in das Leitungsinnere des extrakorporalen Blutkreislaufs und/oder zum Fördern des Fluids darin.

Das Einbringen des zweiten Fluids, im Folgenden vereinfachend - aber nicht einschränkend - als Substituatflüssigkeit bezeichnet, in das Leitungsinnere des extrakorporalen Blutkreislaufs durch Betreiben der zweiten Fördereinrichtung, im Folgenden vereinfachend - aber nicht einschränkend - als Substituatpumpe bezeichnet, erfolgt in bestimmten Ausführungsformen der vorliegenden Erfindung nach Verbinden des ersten Abschnitts des arteriellen Leitungsabschnitts mit dem zweiten Abschnitt des venösen Leitungsabschnitts.

In manchen erfindungsgemäßen Ausführungsformen wird die Blutpumpe derart in der zweiten Förderrichtung betrieben, dass der in das Leitungsinnere des extrakorporalen Blutkreislaufs eingebrachte Substituatflüssigkeitsstrom aufgeteilt wird in wenigstens einen ersten und einen zweiten Teilstrom. Der erste Substituatflüssigkeitsteilstrom bewegt sich zu einer Blutbehandlungseinrichtung hin, der zweite Substituatflüssigkeitsteilstrom bewegt sich in der zweiten Förderrichtung.

In bestimmten Ausführungsformen der vorliegenden Erfindung umfasst das erfindungsgemäße Verfahren das Dekonnektieren des ersten Abschnitts.

In manchen Ausführungsformen der vorliegenden Erfindung umfasst das erfindungsgemäße Verfahren das Konnektieren des ersten Abschnitts mit einer venösen Zugabestelle des venösen Leitungsabschnitts des extrakorporalen Blutkreislaufs oder mit einer venösen Zugabestelle der Funktionseinrichtung (z. B. der Blutkassette).

In bestimmten Ausführungsformen der vorliegenden Erfindung werden die zweite Fördereinrichtung und die Blutpumpe, insbesondere im Wesentlichen oder nahezu oder vollständig, zeitgleich gestartet.

Die Förderraten der zweiten Fördereinrichtung und der Blutpumpe werden oder sind in manchen Ausführungsformen der vorliegenden Erfindung derart eingestellt, dass ein Abschnitt des venösen Leitungsabschnitts, z. B. von einer Prädilutionsstelle oder von einem Prädilutionsventil bis zur venösen Zugabestelle oder bis zu einer Stelle, an welcher sich Fluid aus dem venösen Leitungsabschnitt und Fluid aus dem arteriellen Leitungsabschnitt vereinen oder aufeinandertreffen, nicht eher von Blut geleert wird als ein Abschnitt des arteriellen Leitungsabschnitts, z. B. bis zu dessen Konnektion mit der venösen Zugabestelle oder bis zu jener Stelle, an welcher sich Fluid aus dem venösen Leitungsabschnitt mit Fluid aus dem arteriellen Leitungsabschnitt vereint oder beide Fluide aufeinandertreffen.

In bestimmten Ausführungsformen der vorliegenden Erfindung ist die Förderrate der Blutpumpe (anfangs, stets, oder im Mittel) niedriger als die Förderrate der zweiten Fördereinrichtung oder wird entsprechend eingestellt.

In manchen Ausführungsformen der vorliegenden Erfindung werden die Förderraten der Blutpumpe und/oder der zweiten Fördereinrichtung während des Entfernens von Blut mittels Drucküberwachung und/oder Druckmessung und/oder Druckbegrenzung überwacht und/oder geregelt.

Dabei wird in bestimmten Ausführungsformen der vorliegenden Erfindung die Förderrate $q\_V\_Sub$ der zweiten Fördereinrichtung nach der Formel

$$q\_V\_Sub = q\_V\_BP\left(1 + \frac{V\_ven\_min}{V\_art}\right)$$

$$(\text{I})$$

eingestellt.

In Formel (I) bezeichnet V_ven_min in bestimmten Ausführungsformen der vorliegenden Erfindung das Volumen des venösen Leitungsabschnitts einschließlich des Aufnahmevolumens einer kleinsten Blutbehandlungseinrichtung (beispielsweise bezogen auf verschiedene an einer Blutbehandlungsvorrichtung zu verwendende Blutbehandlungseinrichtungen, siehe unten); q_V_BP bezeichnet die Förderrate der Blutpumpe, V_art das Volumen, welches der arterielle Leitungsabschnitt oder Abschnitt aufnehmen kann.

Die "kleinste Blutbehandlungseinrichtung" ist in bestimmten Ausführungsformen der vorliegenden Erfindung jene Blutbehandlungseinrichtung unter einer Vielzahl von Blutbehandlungseinrichtungen, z. B. Blutfiltern oder Dialysatoren, welche zur Verwendung mit der betrachteten Blutbehandlungsvorrichtung vorbereitet (etwa hinsichtlich der vorhandenen Anschlüsse, Zulassungen, usw.) sind, welche am wenigsten Volumen oder am wenigsten Fluid aufnehmen kann.

Ein typischer Wert kann sich für q_V_Sub wie folgt ergeben zu:

```
q_V_Sub = 50 ml/min (1 + (60 ml/60 ml) = 100 ml.
```

In bestimmten Ausführungsformen der vorliegenden Erfindung wird die Förderrate der zweiten Fördereinrichtung derart eingestellt, dass an der venösen Zugabestelle - oder an einer Stelle, an welcher Fluid aus dem venösen Leitungsabschnitt mit Fluid aus dem arteriellen Leitungsabschnitt vereint wird oder auf dieses trifft - Blut (aus einer Leitung) und Substituatflüssigkeit (aus einer anderen Leitung) gleichen Verdünnungsgrads aufeinandertreffen.

In bestimmten Ausführungsformen der vorliegenden Erfindung umfasst das erfindungsgemäße Verfahren das Einstellen der Förderrate q_V_Sub der zweiten Fördereinrichtung und der Förderrate q_V_BP der Blutpumpe zueinander - individuell für die jeweils verwendete Blutbehandlungseinrichtung - nach der Formel

$$\frac{q\_V\_BP}{q\_V\_Sub} = \frac{V\_art\_S\_h}{V\_Dial \cdot F + V\_art\_S\_h}$$

$$(\text{II})$$

In der Formel (II) gibt V_art_S_h das Substituatvolumen an, welches in den arteriellen Leitungsabschnitt gefördert werden muss, damit sich am Ende des arteriellen Leitungsabschnitts ein Hämatokrit-(HKT-)Wert von ~0,02 einstellt; V_Dial gibt das Volumen der Blutbehandlungseinrichtung, und F einen dimensionslosen Korrekturfaktor an, der das Verhältnis von tatsächlich benötigter Flüssigkeitsmenge zum Freispülen der Blutbehandlungseinrichtung (bis zum Erreichen eines Hämatokrits HKT von etwa 0,02) zu dessen nominellem patientenseitigen Füllvolumen angibt.

V_art_S_h kann einmalig für einen durchschnittlichen Patienten-Hämatokrit experimentell bestimmt werden oder worden sein. Dasselbe gilt für den dimensionslosen Faktor F.

Ein typischer Wert kann sich für q_V_BP/q_V_Sub wie folgt ergeben zu:

```
q_V_BP/q_V_Sub = 60 ml/(80 ml*1,5 + 60 ml) = 1/3.
```

In bestimmten Ausführungsformen des erfindungsgemäßen Verfahrens ist vorgesehen, die Blutpumpe zu stoppen oder ihre Fördergeschwindigkeit oder -rate zu reduzieren, bevor Substituatflüssigkeit stromab (bezogen auf die zweite Förderrichtung oder auf das erfindungsgemäße Verfahren) der venösen Zugabestelle oder der Stelle, an welcher sich Fluid aus dem venösen Leitungsabschnitt und Fluid aus dem arteriellen Leitungsabschnitt vereinen oder aufeinander treffen, gelangt.

Dies kann in bestimmten Ausführungsformen der vorliegenden Erfindung vorteilhaft zu einem stärkeren und/oder verbesserten Durchspülen der Blutbehandlungseinrichtung beitragen.

Der Zeitpunkt zum Stoppen der Blutpumpe oder zum Reduzieren ihrer Förderrate wird in bestimmten Ausführungsformen der vorliegenden Erfindung mit Hilfe des in den extrakorporalen Blutkreislauf integrierten arteriellen Luftblasendetektors/optischen Detektors (auch bekannt als air bubble detector/optical detector, ABD/OD) bestimmt. Dies kann vorteilhaft die Genauigkeit, mit welcher der Zeitpunkt zum Stoppen ermittelt wird, erhöhen.

In anderen Ausführungsformen der vorliegenden Erfindung umfasst das erfindungsgemäße Verfahren das Stoppen der zweiten Fördereinrichtung oder das Reduzieren der Geschwindigkeit oder Förderrate der zweiten Fördereinrichtung, bevor Substituatflüssigkeit stromab (bezogen auf die zweite Förderrichtung oder auf das erfindungsgemäße Verfahren) der venösen Zugabestelle oder der Stelle, an welcher sich Fluid aus dem venösen Leitungsabschnitt und Fluid aus dem arteriellen Leitungsabschnitt vereinen oder aufeinander treffen, gelangt. Dies kann vorteilhaft zu einer stärkeren und/oder verbesserten Durchspülung des arteriellen Leitungsabschnitts des extrakorporalen Blutkreislaufs beitragen.

Die Blutpumpe wird in solchen Ausführungsformen weiter betrieben, oder nicht.

In bestimmten Ausführungsformen der vorliegenden Erfindung umfasst das erfindungsgemäße Verfahren das Überprüfen der Konnektion des ersten Abschnitts, z. B. des arteriellen Nadelanschlusses, des extrakorporalen Blutkreislaufs an der venösen Zugabestelle des venösen Leitungsabschnitts des extrakorporalen Blutkreislaufs (Konnektionstest).

In bestimmten Ausführungsformen der vorliegenden Erfindung umfasst das Überprüfen das Erzeugen eines Druckausgleichs.

In solchen Ausführungsformen kann vorgesehen sein, die Blutpumpe und/oder die zweite Fördereinrichtung zu stoppen. Ferner kann vorgesehen sein, die arterielle Klemme zu öffnen oder offen zu halten.

In bestimmten weiteren Ausführungsformen der vorliegenden Erfindung umfasst die Überprüfung das Bestimmen eines diastolischen Patientendrucks. Ein Minimalwert des diastolischen Patientendrucks kann über einen Zeitraum von z. B. 2,5 s gespeichert werden. Anschließend wird mittels der in der ersten Förderrichtung, also vorwärts, fördernden Blutpumpe ein Unterdruck erzeugt, wobei die venöse Klemme geöffnet wird oder bereits offensteht. In bestimmten Ausführungsformen der vorliegenden Erfindung ist definiert, dass der Gefäßdruck des Patienten innerhalb eines Zeitraums von 2,1 s um 50 mmHg abfallen muss, damit der Konnektionstest als bestanden gilt. Alternativ können andere Werte zu Druckabfall und/oder Zeiten als die hier genannten beachtet werden. Sollte die gewünschte und/oder erforderliche Druckreduktion nicht oder nicht in der vorbestimmten Zeit erfolgt sein, kann der Konnektionstest als nicht bestanden gelten.

Die erfindungsgemäße Funktionseinrichtung ist in einigen erfindungsgemäßen Ausführungsformen als Disposable oder als Wegwerfartikel ausgestaltet.

In bestimmten erfindungsgemäßen Ausführungsformen ist die venöse Zugabestelle der Funktionseinrichtung ausgestaltet, um durch einfaches Verklemmen, Aufstecken oder Verschrauben eines arteriellen Nadelanschlusses oder eines anderen Abschnitts des arteriellen Leitungsabschnitts des extrakorporalen Blutkreislaufs eine Fluidverbindung zu erzeugen.

In manchen erfindungsgemäßen Ausführungsformen sind die venöse Zugabestelle der Funktionseinrichtung und der arterielle Nadelanschluss oder ein anderer Abschnitt des arteriellen Leitungsabschnitts des extrakorporalen Blutkreislaufs vom selben Konnektorsystem, beispielsweise beide Luer-Konnektoren, oder weibliches und männliches Verbindungsstück.

In gewissen erfindungsgemäßen Ausführungsformen ist die Anschlussgeometrie der venösen Zugabestelle gleich oder entsprechend der Anschlussgeometrie einer arteriellen Nadel, mit welcher der extrakorporale Blutkreislauf zu seiner Benutzung verbunden wird, ausgestaltet.

Die erfindungsgemäße Steuereinrichtung ist in einigen erfindungsgemäßen Ausführungsformen als Regeleinrichtung ausgestaltet.

Die erfindungsgemäße Blutbehandlungseinrichtung ist in manchen erfindungsgemäßen Ausführungsformen als Hämodialysevorrichtung oder Hämofiltrationsvorrichtung oder Hämodiafiltrationsvorrichtung ausgestaltet.

Die erfindungsgemäße Blutbehandlungseinrichtung weist in bestimmten erfindungsgemäßen Ausführungsformen wenigstens eine erfindungsgemäße Steuereinrichtung auf.

Manche oder alle erfindungsgemäßen Ausführungsformen können einen, mehrere oder alle der oben und/oder im Folgenden genannten Vorteile aufweisen.

Die vorliegende Erfindung stellt ein einfaches und wenig aufwendiges Verfahren zum Entfernen von Blut aus einem extrakorporalen Blutkreislauf nach einer Blutbehandlungssitzung sowie entsprechende Vorrichtungen bereit. Durch das Entfernen von Blut nach Behandlungsende kann die Gefahr einer Kontaminierung bei der weiteren Handhabung oder Entsorgung des Blutkreislaufs vorteilhaft verringert werden.

In bestimmten erfindungsgemäßen Ausführungsformen erlaubt das Verfahren, nach der Blutbehandlungssitzung im extrakorporalen Blutkreislauf vorhandenes Blut über eine venöse Verbindung mit dem Gefäßsystem des Patienten vollständig an diesen zurückzugeben. Dabei ist es vermeidbar, dem Patienten zugleich Substituatflüssigkeit oder ein anderes Fluid zu infundieren.

Das erfindungsgemäße Verfahren lässt sich bei aus der Praxis bekannten Behandlungsvorrichtungen vorteilhaft bereits allein mittels eines einfach durchzuführenden Software-Updates umsetzen. Die erforderlichen Maschinenelemente weisen aus der Praxis bekannte Blutbehandlungsvorrichtungen häufig bereits auf.

Im Folgenden wird die vorliegende Erfindung unter Bezugnahme auf die beigefügte Figur rein exemplarisch beschrieben. Es gilt:

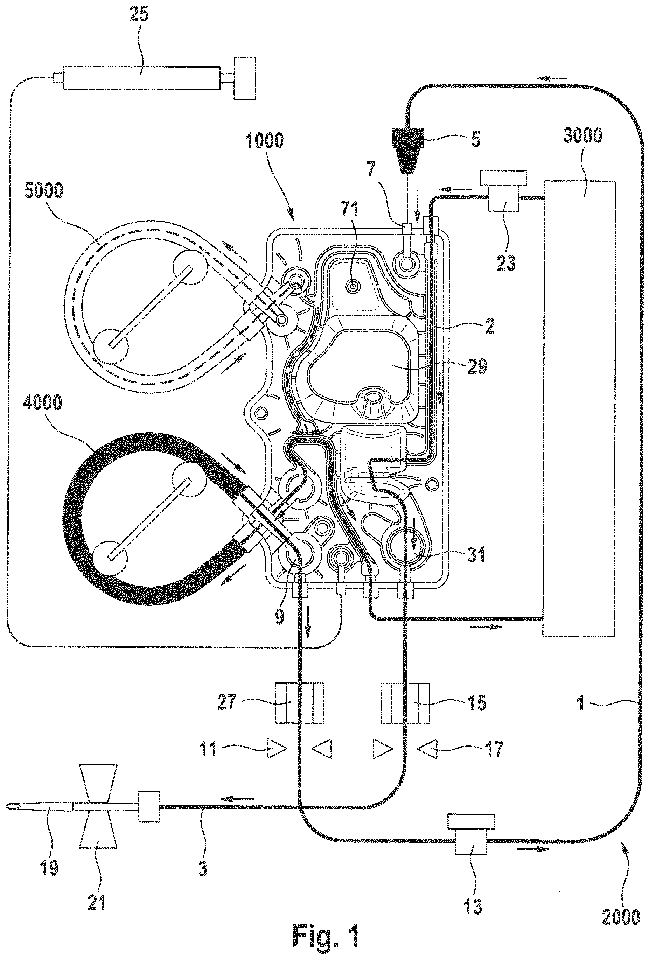

Fig. 1    zeigt in vereinfachter Darstellung eine medizinische Funktionseinrichtung mit einem extrakorporalen Blutkreislauf, welcher mittels des erfindungsgemäßen Verfahrens von Blut befreit werden kann.

Fig. 1 zeigt schematisch vereinfacht eine Funktionseinrichtung 1000 mit einem hieran angeschlossenen extrakorporalen Blutkreislauf 2000.

Der extrakorporale Blutkreislauf 2000 weist eine Blutbehandlungseinrichtung 3000, z. B. einen Blutfilter oder Dialysator, auf oder ist mit dieser verbunden.

Eine in Fig. 1 nur durch einige ihrer Einrichtungen repräsentierte Blutbehandlungsvorrichtung, mittels welcher das hier beschriebene Verfahren ganz oder im Wesentlichen mit einem Reinfundieren des im extrakorporalen Blutkreislauf 2000 enthaltenen Bluts automatisiert abläuft, weist eine Blutpumpe 4000 und eine zweite Fördereinrichtung 5000 auf. Sowohl die Blutpumpe 2000 als auch die zweite Fördereinrichtung 5000 fördern Fluid durch Abschnitte der Funktionseinrichtung 1000 und des extrakorporalen Blutkreislaufs 2000. Die Funktionseinrichtung 1000 ist beispielhaft eine Blutkassette.

Angedeutet sind ein Anschlussschema sowie Flussrichtungen, angegeben durch Pfeile, von Blut und Substituat (als Beispiel eines zweiten Fluids) während der Entfernung des Bluts mit gleichzeitiger Reinfusion des Bluts. Der einzige Doppelpfeil beschreibt eine Aufteilung des Substituatstroms in zwei Teilströme.

Der extrakorporale Blutkreislauf 2000 weist einen arteriellen Leitungsabschnitt 1 sowie einen venösen Leitungsabschnitt 3 auf.

Der arterielle Leitungsabschnitt 1 weist einen ersten Abschnitt auf. Der erste Abschnitt ist im Beispiel der Fig. 1 beispielhaft als arterieller Nadelanschluss 5 ausgestaltet.

Der venöse Leitungsabschnitt 3 weist einen zweiten Abschnitt auf. Der zweite Abschnitt 3 ist in Fig. 1 beispielhaft als venöse Zugabestelle 7 der Funktionseinrichtung 1000 ausgestaltet.

Der arterielle Leitungsabschnitt 1 weist einen arteriellen Drucksensor auf, welcher an der mit Bezugszeichen 9 bezeichneten Stelle an die Funktionseinrichtung 1000 gekoppelt ist ohne selbst Bestandteil der Funktionseinrichtung 1000 zu sein. Dieser Drucksensor dient zum Messen des Drucks oder zur Bestimmung des Druckabfalls während eines Konnektionstests. Er ist im arteriellen Leitungsabschnitt 1 des extrakorporalen Blutkreislaufs 2000 angeordnet.

Der arterielle Leitungsabschnitt 1 weist eine arterielle Klemme 11 auf.

Der arterielle Leitungsabschnitt 1 weist ein arterielles Septum 13 auf.

Der venöse Leitungsabschnitt 3 weist einen venösen Luftblasendetektor/optischen Sensor 15 auf.

Der venöse Leitungsabschnitt 3 weist eine venöse Klemme 17 auf.

Der venöse Leitungsabschnitt 3 weist eine venöse Nadel 19 an einem venösen Patientenkonnektor 21 auf.

Zwischen der Blutbehandlungseinrichtung 3000 und dem Ort seiner Verbindung mit dem auf der Funktionseinrichtung 1000 vorliegenden Abschnitt des extrakorporalen Blutkreislaufs 2000 ist ein venöses Septum 23 angeordnet.

Zur Zugabe von Heparin in das Leitungsinnere des extrakorporalen Blutkreislaufs 2000 während einer extrakorporalen Blutbehandlung ist der extrakorporale Blutkreislauf 2000 über einen entsprechenden Port der Funktionseinrichtung 1000 mit einer Heparinspritze 25 verbunden.

Der arterielle Leitungsabschnitt 3 weist einen arteriellen Luftblasendetektor/optischen Sensor 27 auf.

Bei der Blutbehandlung wird der extrakorporale Blutkreislauf 2000, z. B. wie in Fig. 1 gezeigt in Form eines Schlauchsystems, über zwei Nadeln (im Falle einer Double-Needle-Dialyse), mit dem Gefäßsystem des Patienten verbunden. Zur Durchführung der Blutbehandlung wird der extrakorporale Blutkreislauf 2000 mit Blut des Patienten gefüllt und während der Behandlung durchströmt.

Im Folgenden wird ein erfindungsgemäßes Entfernen von Blut aus dem extrakorporalen Blutkreislauf unter Nutzung der venösen Zugabestelle 7 der Funktionseinrichtung 1000 beschrieben. In dem hier beschriebenen Beispiel einer maschinell durchgeführten Ausführungsform des Verfahrens erfolgt mit der Entfernung von Blut eine gleichzeitige Reinfusion dieses Bluts.

Das erfindungsgemäße Verfahren kann automatisch oder manuell durch den Arzt gestartet werden.

Hierzu wird in bestimmten Ausführungsformen der Erfindung nach Beendigung der Blutbehandlung der arterielle Patientenkonnektor vom arteriellen Nadelanschluss 5 dekonnektiert und mit einem Port der Blutkassette, hier die venöse Zugabestelle 7, in Fluidverbindung verbunden. Wie aus der in Fig. 1 gezeigten Blutkassette zu sehen, kann die venöse Zugabestelle 7 stromaufwärts einer Blutkammer 29 und eines Gerinnselfängers 31 direkt (in anderen Ausführungsformen aber auch indirekt) in den venösen Leitungsabschnitt 3 des extrakorporalen Blutkreislaufs 2000 münden.

Die venöse Zugabestelle 7 ist in den o. g. Anmeldungen der Anmelderin mit den Veröffentlichungsnummern DE 10 2009 018 664 A1 und DE 10 2009 024 468 A1 jeweils in den Fig. 1 und 2 mit dem Bezugszeichen 37 gekennzeichnet. Die venöse Zugabestelle steht über ein Rückschlagventil mit der venösen Filterleitung in Verbindung.

Der Anwender kann zum Ende der Blutbehandlung aufgefordert werden, den arteriellen Nadelanschluss 5 vom arteriellen Patientenkonnektor (in Fig. 1 nicht gezeigt) zu trennen und den arteriellen Nadelanschluss 5 auf den Luer-Konnektor der venösen Zugabestelle 7 der Funktionseinrichtung 1000 zu schrauben. Um zu prüfen, ob die Konnektion korrekt hergestellt ist, kann von der Blutbehandlungsvorrichtung automatisch oder auf Anforderung ein Konnektionstest durchgeführt werden. Dabei wird die korrekte Verbindung des arteriellen Leitungsabschnitts 1 mit dem venösen Leitungsabschnitt 3 geprüft. Ein direktes Fördern des Blutes über den arteriellen Leitungsabschnitt 1 in das Gefäßsystem des Patienten kann somit vorteilhaft vermieden werden.

Der venöse Leitungsabschnitt 3 ist in bestimmten Ausführungsformen mit einem Rückschlagventil versehen, weshalb die Blutpumpe 4000 in der ersten Förderrichtung keine Flüssigkeit aus dem venösen Leitungsabschnitt 3 ansaugen kann. Daher darf erwartet werden, dass bei Durchführen des Konnektionstests der Druck im arteriellen Leitungsabschnitt 1 abnimmt. Nimmt der arterielle Druck wie erwartet ab, kann davon ausgegangen werden, dass der Patient arteriell nicht mehr konnektiert ist, jedenfalls aber die (manuelle) arterielle (Schlauch-)Klemme 11 geschlossen ist.

Ist, wie in bestimmten Ausführungsformen der vorliegenden Erfindung, ein arterieller Druckalarm bei Förderung in diesem Verfahrensstadium vorgesehen, so würde ein Fehler auch ohne Konnektionstest ebenfalls vorteilhaft frühzeitig erkannt werden. Daher kann es vorteilhaft möglich sein, auf ein explizites Testen des Konnektionszustands zu verzichten.

Der Konnektionstest zur Erkennung, ob der arterielle Leitungsabschnitt 1, z. B. mittels des arteriellen Nadelanschlusses 5, mit dem venösen Leitungsabschnitt 3,
z. B. der venösen Zugabestelle 7, verbunden ist, kann dabei im Einzelnen folgendermaßen ablaufen: Zunächst wird ein Druckausgleich hergestellt, wobei die Blutpumpe 4000 und die zweite Fördereinrichtung 5000 gestoppt werden. Die arterielle Klemme 11 ist geöffnet.

Mittels Drucksensor wird der diastolische Patientendruck detektiert. Hierbei wird ein Minimalwert über 2,5 s gespeichert. Anschließend wird ein Unterdruck aufgebaut, wobei die venöse Klemme 17 geöffnet wird und wobei mittels der Blutpumpe 4000 gefördert wird. Der Druck muss innerhalb von beispielsweise 2,1 s um 50 mmHg unter den zuvor detektierten diastolischen Patientendruck abfallen, ansonsten gilt der Konnektionstest als nicht bestanden.

Zum erfindungsgemäßen Entfernen von Blut wird ein Prädilutionsventil oder -anschluss, vorgesehen zur Einleitung von Substituatflüssigkeit in die Blutleitung zwischen der Blutbehandlungseinrichtung 3000 und der Blutpumpe 4000, geöffnet. Die Substituatleitung ist mit dem Prädilutionsanschluss verbunden, so dass die zweite Fördereinrichtung 5000 Substituatlösung in einen zwischen der Blutpumpe 4000 und der Blutbehandlungseinrichtung 3000 gelegenen Abschnitt des extrakorporalen Blutkreislaufs 2000 einleiten kann.

Die Blutpumpe 4000 beginnt in der hier dargestellten Ausführungsform im selben Moment (gleichzeitig), in welchem mittels der zweiten Fördereinrichtung 5000 zu fördern begonnen wird, mit einer geringeren Förderrate rückwärts, d. h. in der zweiten Förderrichtung, zu fördern. Dabei wird der Substituatfluss aufgrund der unterschiedlichen Fördergeschwindigkeiten der beiden Pumpen geteilt - ein Teilstrom der Substituatlösung bewegt sich zur Blutpumpe 4000 hin, ein anderer Teilstrom der Substituatlösung bewegt sich in Richtung auf die Blutbehandlungseinrichtung 3000 zu.

Die sich einstellenden venösen und/oder arteriellen Drücke werden während dieses Prozesses überwacht. Die eingestellten Pumpraten oder Förderraten der Fördereinrichtungen (Blutpumpe 4000 und zweite Fördereinrichtung 5000) können das Entfernen des Bluts entscheidend beeinflussen.

In bestimmten Ausführungsformen der vorliegenden Erfindung wird sichergestellt, dass die Förderrate der Blutpumpe 4000 nicht zu hoch gewählt wird. Damit kann vorteilhaft sichergestellt werden, dass das Blut beim Durchströmen der Einleitungsstelle - beispielsweise mit einem dünneren Schlauch und einem Rückschlagventil versehen - nicht geschädigt wird. Hierzu ist eine Begrenzung der maximalen Förderrate, beispielsweise aufgrund von Erfahrungswerten aus *in-vitro-* bzw. *in-vivo-Tests*, möglich oder vorgesehen.

In manchen Ausführungsformen der vorliegenden Erfindung ist eine Begrenzung und/oder Überwachung des Druckabfalls über der Zugabestelle und/oder der Zugabeleitung und dem Rückschlagventil mit Hilfe des arteriellen Drucksensors 9 beim Reinfundieren möglich.

Beim Einstellen der "venösen" Reinfusionsrate, d. h. der Geschwindigkeit, mit der das extrakorporal vorliegende Blut über den venösen Leitungsabschnitt 3 des extrakorporalen Blutkreislaufs 2000 zum Patienten gefördert wird, wird in manchen erfindungsgemäßen Ausführungsformen sichergestellt, dass jener Teil des extrakorporalen Blutkreislaufs 2000, welcher sich von der Prädilutionsstelle oder dem Prädilutionsventil bis zur venösen Zugabestelle 7 erstreckt, nicht eher oder früher geleert ist, bevor auch der arterielle Leitungsabschnitt 1 des extrakorporalen Blutreislaufs 2000 geleert ist.

Hierdurch kann in bestimmten Ausführungsformen der vorliegenden Erfindung vorteilhaft eine weitere Vermischung von Blut (arteriell) und Substituatflüssigkeit (venös) und eine damit einhergehende unnötige Erhöhung des Reinfusionsvolumens mit den bekannten unangenehmen Folgen für den Patienten verhindert werden.

Da bei einem extrakorporalen Blutkreislauf (auch als Schlauchset bezeichnet) die Volumina der einzelnen Leitungsabschnitte bekannt sind, ist es in bestimmten Ausführungsformen der vorliegenden Erfindung vorgesehen, die maximal mögliche oder zulässige venöse Förderrate als die Förderate im venösen Leitungsabschnitt des extrakorporalen Blutkreislaufs 2000 zu berechnen. Die Berechnung kann wie oben beschrieben erfolgen.

Die venöse Förderrate wird in manchen Ausführungsformen der vorliegenden Erfindung eingestellt durch die Förderrate q_V_Sub der zweiten Fördereinrichtung 5000 ("Substituatpumpe") abzüglich der Förderrate q_V_BP der Blutpumpe 4000. Hierzu wird auf die obenstehenden Formeln verwiesen.

Da bei einer solchen fixen Festlegung des Volumens der Blutbehandlungseinrichtung 3000 der arterielle Leitungsabschnitt 1 des extrakorporalen Blutkreislaufs 2000 früher oder schneller als der venöse Leitungsabschnitt 3 geleert werden kann, wird in bestimmten Ausführungsformen der vorliegenden Erfindung vorgeschlagen, die Blutpumpe 4000 zu stoppen, bevor Substituatflüssigkeit über die venöse Zugabestelle 7 hinweg gefördert wird.

Ein solches Stoppen der Förderung mittels der Blutpumpe 4000 ist in bestimmten Ausführungsformen bei Kenntnis des o. g. Volumens mittels entsprechenden Einstellens der Förderrate der Blutpumpe 4000 möglich. Ein - ggf. vorteilhaft noch genaueres - Abpassen des Moments, zu welchem die Blutpumpe 4000 gestoppt werden kann, ist auch unter Berücksichtigung der Signale des arteriellen Luftblasendetektors oder optischen Sensors 11 - soweit vorhanden - möglich.

In einigen erfindungsgemäßen Ausführungsformen wird mit dem Vorteil der Zeitersparnis ab dem Zeitpunkt des Anhaltens der Blutpumpe 4000 die Förderrate der zweiten Fördereinrichtung 5000 erhöht.

Ist das Volumen der jeweils eingesetzten Blutbehandlungseinrichtung 3000 bekannt, wird die Förderrate vorteilhaft bei jeder Reinfusion individuell auf die maximal mögliche Förderrate eingestellt. Dies verkürzt aufgrund der zügiger ablaufenden Entfernung von Blut aus dem extrakorporalen Blutkreislauf 2000 die Zeitdauer, die der Patient und das Bedienpersonal bis zum Abschluss dieser Maßnahme an der Blutbehandlungsvorrichtung verbringen müssen.

Der Typ der verwendeten Blutbehandlungseinrichtung 3000 kann vom Bedienpersonal eingestellt werden. Alternativ kann anhand bestimmter Parameter, welche beim Füllen der Blutbehandlungseinrichtung 3000 zu beobachten sind, der verwendete Typ automatisch ermittelt werden.

Ist das Volumen der Blutbehandlungseinrichtung 3000 bekannt, so wird in einigen erfindungsgemäßen Ausführungsformen die Förderrate der zweiten Fördereinrichtung 5000 derart eingestellt, dass an der Einleitstelle der venösen Zugabestelle 7 (oder einer vergleichbaren Stelle im Blutkreislauf) Substituat und Blut gleichen Verdünnungsgrades aus beiden Leitungsabschnitten 1, 3 zeitgleich aufeinandertrifft. Die Blutpumpe 4000 kann anschließend entweder angehalten werden; alternativ läuft sie weiter.

In Ausführungsformen, in denen die Blutpumpe 4000 weiterläuft wird vorteilhaft der arterielle Leitungsabschnitt 1 des extrakorporalen Blutkreislaufs 2000 vergleichsweise besser durchspült; in Ausführungsformen, in denen die Blutpumpe 4000 gestoppt wird, wird vorteilhaft die Blutbehandlungseinrichtung 3000 vergleichsweise besser durchspült.

Die individuelle Einstellung der Förderraten geschieht in bestimmten Ausführungsformen gemäß der o. g. Formel (II).

**Bezugszeichenliste**

| Bezugszeichen | Bezeichnung |
| --- | --- |
| 1000 | Funktionseinrichtung |
| 2000 | extrakorporaler Blutkreislauf |
| 3000 | Blutbehandlungseinrichtung |
| 4000 | Blutpumpe |
| 5000 | zweite Fördereinrichtung |
| 1 | arterielle Leitungsabschnitt |
| 3 | venöser Leitungsabschnitt |
| 5 | arterieller Nadelanschluss |
| 7 | venöse Zugabestelle |
| 9 | (arterieller) Drucksensor |
| 11 | arterielle Klemme |
| 13 | arterielles Septum |
| 15 | venöser Luftblasendetektor/optischer Sensor |
| 17 | venöse Klemme |
| 19 | venöse Nadel |
| 21 | venöser Patientenkonnektor |
| 23 | venöses Septum |

(fortgesetzt)

| Bezugszeichen | Bezeichnung |
|---|---|
| 25 | Heparinspritze |
| 27 | arterieller Luftblasendetektor/optischer Sensor |
| 29 | Blutkammer |
| 31 | Gerinnselfänger |

**Patentansprüche**

1. Funktionseinrichtung (1000), insbesondere Blutkassette, mit einem venösen Leitungsabschnitt und einer hiermit in Verbindung stehenden venösen Zugabestelle (7), wobei die Zugabestelle (7) mit einem arteriellen Nadelanschluss (5) des arteriellen Leitungsabschnitts (1) eines extrakorporalen Blutkreislaufs (2000) verbunden ist.

2. Set mit wenigstens einer Funktionseinrichtung (1000) gemäß Anspruch 1 und wenigstens einem extrakorporalen Blutkreislauf (2000), wobei der arterielle Nadelanschluss (5) oder ein anderer Abschnitt des arteriellen Leitungsabschnitts (1) des extrakorporalen Blutkreislaufs (2000) mit der venösen Zugabestelle (7) der Funktionseinrichtung (1000) verbunden ist.

3. Blutbehandlungsvorrichtung, insbesondere als Hämodialysevorrichtung, Hämofiltrationsvorrichtung oder Hämodiafiltrationsvorrichtung ausgestaltet, mit einer Steuer- oder Regelungseinrichtung, konfiguriert zur Durchführung eines Verfahrens zum Entfernen von Blut aus einem extrakorporalen Blutkreislauf (2000) und/oder einer Funktionseinrichtung (1000), welche jeweils zum Zweck einer Blutbehandlung eines Patienten mit der Blutbehandlungsvorrichtung verbunden ist bzw. sind, nach Beenden der Blutbehandlungssitzung,
wobei die Blutbehandlungsvorrichtung aufweist oder verbunden ist mit:

   - wenigstens einen/m extrakorporalen Blutkreislauf (2000) mit einem Leitungsinneren, wobei der extrakorporale Blutkreislauf (2000) wenigstens einen arteriellen Leitungsabschnitt (1) und wenigstens einen venösen Leitungsabschnitt (3) aufweist;
   - wenigstens eine/r Blutpumpe (4000) zum Fördern von Blut innerhalb des Leitungsinneren;

   wobei das Verfahren den Schritt umfasst:

   - Betreiben der Blutpumpe (4000) in einer zur während der Blutbehandlung üblichen ersten Förderrichtung dieser Pumpe entgegengesetzten zweiten Förderrichtung;

   wobei ein erster Abschnitt des arteriellen Leitungsabschnitts (1) mit einem zweiten Abschnitt des venösen Leitungsabschnitts (3) des extrakorporalen Blutkreislaufs (2000) verbunden ist.

4. Blutbehandlungsvorrichtung nach Anspruch 3, wobei die Blutbehandlungsvorrichtung wenigstens eine zweite Fördereinrichtung (5000), insbesondere eine Substituatpumpe, zum Einbringen eines zweiten Fluids, insbesondere einer Substituatflüssigkeit, in das Leitungsinnere des extrakorporalen Blutkreislaufs (2000) und/oder zum Fördern eines Leitungsinhalts innerhalb des Leitungsinneren des extrakorporalen
Blutkreislaufs (2000) aufweist; und
wobei das Verfahren den Schritt umfasst:

   - Einbringen des zweiten Fluids, insbesondere der Substituatflüssigkeit, in das Leitungsinnere des extrakorporalen Blutkreislaufs (2000) durch Betreiben der zweiten Fördereinrichtung (5000) nachdem der erste Abschnitt des arteriellen Leitungsabschnitts (1) mit einem zweiten Abschnitt des venösen Leitungsabschnitts (3) verbunden wurde.

5. Blutbehandlungsvorrichtung nach einem der Ansprüche 3 bis 4, wobei die zweite Fördereinrichtung (5000) und die Blutpumpe (4000) zeitgleich gestartet werden.

6. Blutbehandlungsvorrichtung nach einem der Ansprüche 3 bis 5, wobei die Förderraten (q_V_BP, q_V_Sub) der Blutpumpe (4000) und der zweiten
Fördereinrichtung (5000) derart eingestellt werden, dass der venöse Leitungsabschnitt (3) nicht vor dem arteriellen
Leitungsabschnitt (1) geleert wird.

7. Blutbehandlungsvorrichtung nach einem der Ansprüche 3 bis 6, wobei die Förderrate (q_V_BP) der Blutpumpe (4000) kleiner als die Förderrate (q_V_Sub) der zweiten Fördereinrichtung (5000) ist.

8. Blutbehandlungsvorrichtung nach einem der Ansprüche3 bis 7, wobei die Förderraten (q_V_BP, q_V_Sub) der Blutpumpe (4000) und der zweiten
Fördereinrichtung (5000) während des Entfernens von Blut mittels Drucküberwachung und/oder Druckmessung und/oder Druckbegrenzung überwacht und/oder geregelt werden.

9. Blutbehandlungsvorrichtung nach einem der Ansprüche 3 bis 8, wobei die Förderrate (q_V_Sub) der zweiten Fördereinrichtung (5000) nach der Formel (I)

$$q\_V\_Sub = q\_V\_BP\left(1 + \frac{V\_ven\_\min}{V\_art}\right)$$

eingestellt wird,
worin

V_ven_min das Volumen des venösen Leitungsabschnitts (3) bei einer minimalen oder kleinsten Blutbehandlungseinrichtung (3000) angibt; und wobei
V_art das Volumen angibt, welches der arterielle Leitungsabschnitt (1) aufnehmen kann.

10. Blutbehandlungsvorrichtung nach einem der Ansprüche 3 bis 9, wobei das Verfahren den Schritt umfasst:

- Einstellen der Förderrate (q_V_Sub) der zweiten Fördereinrichtung (5000) derart, dass an der venösen Zugabestelle (7) oder an einer Stelle, an welcher sich Fluid aus dem venösen Leitungsabschnitt und Fluid aus dem arteriellen Leitungsabschnitt vereinen oder aufeinandertreffen, Blut und Substituatflüssigkeit gleichen Verdünnungsgrads aufeinander treffen.

11. Blutbehandlungsvorrichtung nach einem der Ansprüche 3 bis 10, wobei das Verfahren den Schritt umfasst:

- Einstellen der Förderraten (q_V_BP, q_V_Sub) der Blutpumpe (4000) und der zweiten Fördereinrichtung (5000) - insbesondere individuell für die jeweils verwendete Blutbehandlungseinrichtung (3000) - nach der Formel (II)

$$\frac{q\_V\_BP}{q\_V\_Sub} = \frac{V\_art\_S\_h}{V\_Dial \cdot F + V\_art\_S\_h}$$

worin

V_art_S_h das Substituatvolumen angibt, welches in den arteriellen Leitungsabschnitt (1) gefördert werden muss, damit sich am Ende des arteriellen Leitungsabschnitts (1) ein Hämatokrit-(HKT-)Wert von ~0,02 einstellt;
V_Dial das Volumen der Blutbehandlungseinrichtung (3000) angibt; und
F einen dimensionslosen Korrekturfaktor angibt, der das Verhältnis von tatsächlich benötigter Flüssigkeitsmenge zum Freispülen der Blutbehandlungseinrichtung (3000) (bis HKT ~0,02) zu dessen nominellem patientenseitigen Füllvolumen angibt.

12. Blutbehandlungsvorrichtung nach Anspruch 11, wobei V_art_S_h und/oder der Faktor F einmalig für einen durchschnittlichen Patienten-Hämatokrit (HKT) experimentell bestimmt werden oder wurden.

13. Blutbehandlungsvorrichtung nach einem der Ansprüche 3 bis 12, wobei das Verfahren den Schritt umfasst:

- Stoppen der Blutpumpe (4000), bevor Substituatflüssigkeit an der venösen Zugabestelle (7) in den venösen Leitungsabschnitt (3) des extrakorporalen Blutkreislaufs (2000) eingebracht wird, oder bevor Substituatflüssigkeit eine Stelle erreicht, an welcher sich Fluid aus dem venösen Leitungsabschnitt und Fluid aus dem arteriellen Leitungsabschnitt miteinander vereinen oder aufeinandertreffen;

oder

- Stoppen der zweiten Fördereinrichtung (5000) bevor Substituatflüssigkeit an der venösen Zugabestelle (7) in den venösen Leitungsabschnitt (3) des extrakorporalen Blutkreislaufs (2000) eingebracht wird, oder bevor Substituatflüssigkeit eine Stelle erreicht, an welcher sich Fluid aus dem venösen Leitungsabschnitt und Fluid aus dem arteriellen Leitungsabschnitt miteinander vereinen oder aufeinandertreffen.

14. Blutbehandlungsvorrichtung nach einem der Ansprüche 3 bis 13, das Verfahren ferner umfassend den Schritt:

- Überprüfen der Konnektion des ersten Abschnitts des arteriellen Leitungsabschnitts (1), insbesondere des arteriellen Nadelanschlusses (5), des extrakorporalen Blutkreislaufs (2000) mit dem zweiten Abschnitt des venösen Leitungsabschnitts (3), insbesondere der venösen Zugabestelle (7), des extrakorporalen Blutkreislaufs (2000).

15. Blutbehandlungsvorrichtung nach Anspruch 14, wobei die Überprüfung umfasst:

- Erzeugen eines Druckausgleichs;
- Detektieren eines diastolischen Patientendrucks; und
- Aufbauen eines Unterdrucks mittels der in erster Förderrichtung oder vorwärts fördernden Blutpumpe (4000).

16. Blutbehandlungsvorrichtung nach einem der Ansprüche 3 bis 15, wobei die venöse Zugabestelle (7) des venösen Leitungsabschnitts (3) des extrakorporalen Blutkreislaufs (2000) stromaufwärts einer Blutkammer (29) und stromaufwärts eines Gerinnselfängers (31) in den venösen Leitungsabschnitt (3) mündet.

**Fig. 1**

**IN DER BESCHREIBUNG AUFGEFÜHRTE DOKUMENTE**

**In der Beschreibung aufgeführte Patentdokumente**

- DE 102009018664 A1
- DE 102009024468 A1